# EUROPÄISCHE PATENTSCHRIFT

(11) **EP 1 528 918 B1**
(45) Veröffentlichungstag und Bekanntmachung des Hinweises auf die Patenterteilung: **30.03.2016**
(21) Anmeldenummer: 03790867.0
(22) Anmeldetag: 06.08.2003
(51) Int. Cl.: A61K 9/70, A61K 47/32

(54) **HAUTFREUNDLICHES WIRKSTOFFPFLASTER AUF DER BASIS VON SBC MIT MINDESTENS EINEM PHARMAZEUTISCHEN WIRKSTOFF MIT EINEM GEHALT VON MINDESTENS 34 GEW.-% UND DESSEN HERSTELLUNG**
SKIN FRIENDLY ACTIVE INGREDIENT PLASTER BASED ON SBC, CONTAINING AT LEAST 34 WT % OF A PHARMACEUTICAL ACTIVE INGREDIENT AND PRODUCTION THEREOF
TIMBRE DE SUBSTANCES ACTIVES DOUX POUR LA PEAU, A BASE DE SBC, COMPRENANT AU MOINS 34 % EN POIDS D'UNE SUBSTANCE ACTIVE PHARMACEUTIQUE ET PROCEDE POUR PRODUIRE CE TIMBRE

(30) Priorität: 08.08.2002 DE 10236319
(43) Veröffentlichungstag der Anmeldung: 11.05.2005
(73) Patentinhaber: Beiersdorf AG, 20245 Hamburg (DE)
(72) Erfinder: KUMMER, Andreas, 21079 Hamburg (DE); WASNER, Matthias, 21039 Börnsen (DE); WUESTLING, Jens-Uwe, 22589 Hamburg (DE)
(86) Internationale Anmeldenummer: PCT/EP2003/008709
(87) Internationale Veröffentlichungsnummer: WO 2004/019919

(56) Entgegenhaltungen:
- EP-A- 1 116 763
- WO-A-02/03970
- WO-A-98/34600
- WO-A-03/011260
- US-A- 5 580 572
- US-A- 5 603 948
- US-A- 6 007 835

## Beschreibung

Die Erfindung betrifft ein wirkstoffhaltiges Pflastersystem zur kontrollierten Abgabe von mindestens einem pharmazeutischen Wirkstoff, enthaltend mindestens 34 Gew.-%, an die Haut über einen längeren Zeitraum sowie ein Verfahren zu seiner Herstellung.

Transdermal Therapeutische Systeme (TTS) zur Abgabe von Wirkstoffen durch die Haut sind seit langer Zeit bekannt. Die topische Applikation von Arzneimitteln über wirkstoffhaltige Pflastersysteme bietet zwei Hauptvorteile: Erstens wird durch diese Darreichungsform eine Freisetzungskinetik des Wirkstoffes erster Ordnung realisiert, wodurch über einen sehr langen Zeitraum ein konstanter Wirkstoffspiegel im Organismus aufrechterhalten werden kann. Zweitens werden über den Aufnahmeweg durch die Haut der Magen-Darm-Trakt sowie die erste Leberpassage vermieden. Dadurch können ausgewählte Arzneistoffe in einer geringen Dosierung wirkungsvoll verabreicht werden. Dies ist insbesondere dann von Vorteil, wenn eine lokale Wirkung des Arzneistoffes unter Umgehung einer systemischen Wirkung erwünscht ist. Dies ist zum Beispiel bei der-Behandlung von Hyperkeratosen, Hühneraugen, Schwielen und Warzen der Fall.

Eine in der Fachliteratur gut beschriebene Ausführungsform solcher transdermalen Systeme stellen Matrixsysteme oder monolitische Systeme dar, in denen der Arzneistoff direkt in den druckempfindlichen Haftklebstoff eingearbeitet wird. Eine solche haftklebrige, wirkstoffhaltige Matrix ist im anwendungsfertigen Produkt auf der einen Seite mit einem für den Wirkstoff undurchlässigen Träger ausgestattet, auf der gegenüberliegenden Seite befindet sich eine mit einer Trennschicht ausgestatten Trägerfolie, die vor der Applikation auf die Haut entfernt wird (kleben & dichten, Nr.42, 1998, S. 26 bis 30).

Eine Grundanforderung an ein TTS ist einerseits ein gutes Haftvermögen auf Haut, das über den gesamten Zeitraum der beabsichtigten Wirkstoffdosierung aufrechterhalten bleiben muss, und andererseits eine rückstandsfreie Entfernbarkeit des TTS. Auch ein schmerzhaftes Wiederablösen des wirkstoffhaltigen Pflasters nach längerer Tragezeit wird häufig beobachtet. Neben Klebmassen, die in Lösung auf den Träger beschichtet werden, kommen u.a. auch lösungsmittelfreie Systeme, wie Heißschmelzklebmassen zum Einsatz. Diese zeichnen sich dadurch aus, dass bei der Beschichtung auf die Verwendung von organischen Lösungs- und Dispergiermittel verzichtet werden kann. Heißschmelzklebmassen werden durch Erwärmen in eine flüssige Form überführt und so als Schmelze auf den jeweiligen Pflasterträger aufgebracht. Neben technischen Gesichtspunkten, wie Lösungsmittelaufbereitung, Ausführungen der Anlagen mit Explosionsschutz und Umweltschutzauflagen, spielen auch medizinische Gründe für die Wahl von lösungsmittelfreien Klebstoffen eine Rolle. Transdermale therapeutische Systeme werden in der Regel auf gesunder, intakter Haut appliziert. Gerade hier ist es besonders wichtig, die Haut nicht durch ein Arzneimittel zu reizen oder gar zu schädigen. Eine häufig beobachtete Nebenwirkung ist dabei das Auftreten von Hautirritationen, die besonders bei längerer oder wiederholter Applikation eines TTS an einer gleichbleibenden Körperregion auftreten. Sie werden hauptsächlich durch die Inhaltsstoffe der haftklebrigen Matrix verursacht. Bei lösungsmittelhaltigen Systemen lässt sich nach dem Extrahieren der Lösungsmittel ein Restgehalt wiederfinden, der aufgrund des allergenen Potenzials ebenfalls zu unerwünschten Hautirritationen führen kann. Vor allem für die Anwendung auf Haut sind daher lösungsmittelfreie Systeme vorzuziehen, deren Formulierungen insbesondere hautfreundliche Inhaltstoffe beinhalten.

Beschrieben werden Heißschmelzklebmassen in EP-A 663431, EP-A 452 034, EP-A 305 757, DE-A 42 22 334 und DE-C 4224325. Als Wirkstoffe werden in diesen Schriften, wenn diese benannt werden, systemisch wirkende aufgeführt. Systemisch wirkende TTS zeichnen sich dadurch aus, dass sie den Wirkstoff durch die Haut in die Blutbahn geben. Das Blut fördert den Wirkstoff über den gesamten Körper an den Wirkort. Lokal wirksame TTS wirken nur an der applizierten Stelle, da die Haut für den Wirkstoff nicht passierbar ist. Anwendung finden lokal wirksame Systeme u.a. in Wärmetherapien, zur Haut- und Fußpflege.

WO 03011260 A1 offenbart ein Verfahren zur kontinuierlichen Herstellung und Beschichtung von Selbstklebemassen auf Basis von Styrolblockcopolymneren (SBC) mit mindestens einem pharmazeutischen Wirkstoff. Der Wirkstoffanteil im Pflastersystern kann im Bereich zwischen 0,001 bis 0,7 Gew.% gewählt werden.

WO 0203970 A1 beschreibt wirkstoffhaltige transdermale therapeutische Systeme zur Anwendung von stabilen Derivaten von ACE-Hemmern, deren Metabolite eine Dicarbonsäure darstellen. Als Matrixgrundlage dieser Systeme werden übliche Matrixbildner, wie Polyacrylat, Polyisobutylen, Kautschuk, kautschukähnliche synthetische Homo-, Co- oder Blockpolymere, Styrol/Butadien-Copolymerisate oder ein Gemisch daraus, bevorzugt Polyacrylate und Polyisobutylene, beschrieben. Der Wirkstoffanteil beträgt bis zu 10 Gew.%.

WO 9834600 A1 offenbart wirkstoffhaltige transdermale therapeutische Systeme mit einer nicht klebrigen Rückschicht, einer wirkstoffhaltigen Reservoirschicht, einer der Haut zugewandten selbstklebenden Oberfläche und einer elastischen, durch Schichtwachstum gebildeten Metallschicht als für Wirkstoffe Durchlassbarriere.
Die Reservoirschicht kann aus Styrol-Isopren-Styrol-Copolymeren gebildet werden.

EP 1116763 A2 beschreibt ein Verfahren zur kontinuierlichen lösungsmittelfreien und mastikationsfreien Herstellung von Selbstklebemassen auf Basis von Polyisobutylen mit mindestens einem pharmazeutischen Wirkstoff. Der pharmazeutische Wirkstoffanteil in der Selbstklebemasse kann zwischen 0,001 Gew.% und 60 Gew % betragen

US 5580572 beschreibt ein transdermales Matrixsystem auf Basis eines Styrol-Isopren Copolymers zur Verabreichung eines Hormons mit einem Wirkstoffanteil von bis zu 5 Gew.%.

US 5603948 beschreibt ein Wirkstoffpflaster für die kontrollierte Abgabe leichtflüchtiger Wirkstoffe an die Haut. Als Matrix sind Styrolblockcopolymere beschrieben und der Wirkstoffanteil beträgt maximal 25 Gew.%.

US 6007835 beschreibt ein transdermales Matrixsystem auf Basis eines Styrol-Isopren Copolymers zur Verabreichung eines Hormons mit einem Wirkstoffanteil von bis zu 12 Gew.%.

Zur Herstellung von Haftschmelzklebstoffen sind u.a. SBC-Polymere als Gerüstsubstanz bekannt. Beschrieben wird ein derartiges System in DE 196 50 471 A1 zur Applikation von hyperämisierenden Wirkstoffen. Hyperämisierende Wirkstoffe, wie Capsaicin, Nonylsäurevanillylamid oder Belladonna zeichnen sich dadurch aus, dass bereits geringe Menge für die gezielte Wärmetherapie ausreichen. Anwendungen in Konzentrationen oberhalb von 20 Gew-% sind für diese Wirkstoffsysteme nicht bekannt.

Ziel der vorliegenden Erfindung ist es, ein hautfreundliches wirkstoffhaltiges Pflastersystem zur kontrollierten Abgabe mindestens eines pharmazeutischen Wirkstoffes in Konzentrationen von mindestens 34 Gew-% an die Haut zu entwickeln, das den genannten Ansprüchen an ein TTS gerecht wird.

Gelöst wird die Aufgabe durch wirkstoffhaltige Pflaster gemäß Anspruch 1 bzw. 17. Des weiteren umfasst die Erfindung Verfahren zur Herstellung derartiger Pflaster.

Es hat sich gezeigt, dass SBC-Polymere den gestellten Ansprüchen gerecht werden. Die Heißschmelzklebmassen auf Basis von SBC-Polymeren sind durch ihre synthetische Herstellung frei von unerwünschten Inhaltsstoffen und zeichnen sich je nach Molekulargewicht durch eine mit Hilfe von Tackifiern wie Harzen, Ölen und Weichmachern einstellbares Adhäsions- und Kohäsionsvermögen aus. Durch ihr thermoplastisches Verhalten lassen sie sich in der Schmelze mastikationsfrei verarbeiten. Die thermoplastischen Eigenschaften der SBC-Polymere verhelfen auch zu einer Wirkstoffdotierung mit hoher. Wiederfindungsrate, da sich Wirkstoffe auch in hohen Konzentrationen bei relativ geringen Temperaturen und Mischzeiten in der Klebmasseschmelze homogen verteilen lassen und durch zügiges Abkühlen der Abbau thermisch empfindlicher Wirkstoffe im Vergleich zu Lösungsmittelsystemen reduziert werden kann, da lange Trocknungszeiten zum Verdampfen der Dispergier- und/oder Lösungsmittel entfallen.

Die SBC-Polymere sind bevorzugt A-B- oder A-B-A-Blockcopolymer oder deren Mischungen, wobei die harte Phase A vornehmlich Polystyrol oder dessen Derivate und die weiche Phase B Ethylen, Propylen, Butylen, Butadien, Isopren oder deren Mischungen enthält. Polystyrolblöcke können aber auch in Konzentrationen bis zu 20 % in der weichen Phase vorhanden sein.

Demgemäß betrifft die Erfindung bevorzugt ein wirkstoffhaltiges Matrixpflaster zur kontrollierten Abgabe von Wirkstoffen an die Haut, bestehend aus einer flexiblen Deckschicht und einer wirkstoffhaltigen, wasserunlöslichen haftklebrigen Matrix, wobei die haftklebrige Matrix aufgebaut ist aus:

| | |
|---|---|
| A | Blockcopolymeren zu 4 bis 55 Gew.-%, |
| B | Klebrigmachern zu 5 bis 55 Gew.-%, |
| C | Weichmachern zu weniger als 51 Gew.-%, |
| D | Stabilisatoren zu weniger als 5 Gew.-%, |
| E | Wirkstoffen zu 34 bis 70 Gew.-% |

Eine typischer erfindungsgemäßer Haftklebstoff besteht somit aus folgenden Komponenten:

| | | |
|---|---|---|
| A | 5 - 50 Gew. % | bevorzugt 7 - 45 Gew. % |
| B | 7 - 52 Gew.-% | bevorzugt 10 - 50 Gew. % |
| C | 2 - 50 Gew.-% | bevorzugt 4 - 40 Gew. % |
| D | 0 - 5 Gew.-% | bevorzugt 0,2 - 3 Gew. % |
| E | 34 - 70 Gew.-% | bevorzugt 36 - 65 Gew.-% |
| | | besonders bevorzugt 37 - 62 Gew.-% |

Optional können noch bis zu 20 Gew.-% eines permeationsfördernden Hilfsstoffes zugesetzt werden.

Die genannten Rezepturbestandteile werden dabei wie folgt genauer definiert:
A:
   Phasenseparierende Blockcopolymere, insbesondere A-B oder A-B-A-Blockcopolymere oder deren Mischungen auf der Basis von Poly(styrol-b-isopren-b-styrol) (SIS), Poly(styrol-b-isopren-b-styrol) (SBS) oder Poly[styrol-b-(ethylen-stat.-butylen)-b-styrol] (SEBS). Der gesamte Styrolanteil sollte dabei stets niedriger als 35 Gew.-% liegen. Bevorzugt werden Styrolanteile zwischen 5% und 30%, da ein niedrigerer Styrolanteil die Klebemasse anschmiegsamer macht. Insbesondere die gezielte Abmischung von Di-Block- und Tri-Block-copolymeren ist vorteilhaft, wobei ein Anteil an Di-Blockcopolymeren von kleiner 80 Gew.-% bevorzugt wird.
B:
   Klebrigmacher, beispielsweise alle bekannten klebrigmachenden Polymere, z.B. aus der Gruppe der Polyisoprene, Polybutadiene und Polyacrylate sowie klebrigmachende Harze. Hier sind ausnahmslos alle vorbekannten und in der Literatur beschriebenen Klebharze einsetzbar. Genannt seien stellvertretend die Kolophoniumharze, deren disproportionierte, hydrierte, polymerisierte, veresterte Derivate und Salze, die aliphatischen und aromatischen Kohlenwasserstoffharze, Terpenharze und Terpenphenolharze. Beliebige Kombinationen dieser und weiterer Harze können eingesetzt werden, um die Eigenschaften der resultierenden Klebmasse wunschgemäß einzustellen. Auf die Darstellung des Wissensstandes im "Handbook of Pressure Sensitive Adhesive Technology" von Donatas Satas (van Nostrand, 1999) sei ausdrücklich hingewiesen.
C:
   Weichmacher, alle bekannten weichmachenden Substanzen sowie pharmazeutische Hilfsstoffe. Dazu zählen unter anderem die paraffinischen und naphthenischen Öle, (funktionalisierte) Oligomere wie Oligobutadiene, -isoprene, flüssige Nitrilkautschuke, flüssige Terpenharze, pflanzliche und tierische Öle und Fette, Fettsäureester, Phthalate, Alkohole und funktionalisierte Acrylate.
D:
   Stabilisatoren, alle bekannten Substanzen, die die thermische oder oxidative Degradation der wirkstoffhaltigen, wasserunlöslichen haftklebrigen Matrix verhindern oder verzögern. Dieses können bespielsweise Vertreter aus den Gruppen der sterisch gehinderten Phenole, der thioorganischen Verbindungen, hier bevorzugt der Derivate der Thiopropionsäure, der phosphorhaltigen Verbindungsklassen Phosphite und Phosphonite oder deren Mischungen sein.
E:
   Als pharmazeutische Wirkstoffe werden Substanzen verstanden, die in menschlichen oder tierischen Organismen zur Verhütung, Heilung, Linderung oder Erkennung von Krankheiten dienen. Die eingesetzten pharmazeutischen Wirkstoffe können sowohl systemisch als auch lokal wirksam sein. Bevorzugt werden topisch wirkende Wirkstoffe eingesetzt.

Beispielhaft sei hier die Salicylsäure (2-Hydroxybenzoesäure) genannt.

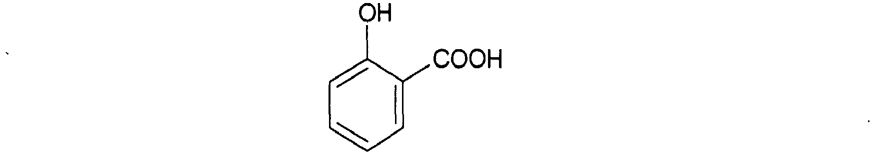

Bevorzugt erfolgt die Herstellung der wirkstoffhaltigen Matrix in einem Verfahren, bei dem alle Komponenten der haftklebrigen Matrix unter Verzicht auf den Zusatz von Lösungsmittel in der Schmelze homogenisiert werden.

Der Vorteil des Lösungsmittelverzichts besteht im wesentlichen in der Vereinfachung des Streichprozesses. Der Verzicht auf brennbare Lösemittel macht die Trockneranlage mit ihrem aufwendigen Energieaufwand für das Verdampfen und Rückgewinnen der Lösemittel und den Einsatz explosionsgeschützter Anlagen überflüssig. Hotmeltbeschichtungsanlagen sind kompakt und erlauben erheblich höhere Streichgeschwindigkeiten. Es handelt sich um eine umweltfreundliche Technologie, bei der keine Lösemittelemissionen auftreten. Weiterhin verbleiben so keine unerwünschten Lösungsmittelreste in der Selbstklebemasse. Dieses begründet die Senkung des allergenen Potenzials des Produkts.

Für die lösungsmittelfreie Compoundierung werden gemäß dem Stande der Technik vorwiegend Blockcopolymere mit Polystyrolblockanteilen oder Natur- bzw. Synthesekautschuke verwendet.

Durch die hochmolekularen Anteile im Naturkautschuk (mit M_{w} ≥ 3*10⁵ g/mol) sind lösungsmittelfreie Naturkautschuk-Selbstklebemassen mit der Schmelzhaftklebertechnologie unverarbeitbar oder aber die verwendeten Kautschuke müssen vor der Verarbeitung stark in ihrem Molekulargewicht reduziert (abgebaut) werden.

Der zielgerichtet durchgeführte technische Prozess des Kautschukabbaus unter der kombinierten Einwirkung von Scherspannung, Temperatur, Luftsauerstoff wird in der Fachliteratur als Mastikation (engl.: mastication) bezeichnet und zumeist im Beisein chemischer Hilfsmittel durchgeführt, welche aus der Fachliteratur als Mastiziermittel oder Peptizer, seltener auch als "chemische Plastiziermittel" bekannt sind.

Der Mastikationsschritt ist in der Kautschuktechnologie nötig, um eine Erleichterung der Aufnahme der Zusatzstoffe zu erzielen.

Nach Römpp (Römpp Lexikon Chemie - Version 2.0, Stuttgart/New York: Georg Thieme Verlag 1999) stellt die Mastikation eine gummitechnologische Bezeichnung für den Abbau langkettiger Kautschuk-Moleküle zur Erhöhung der Plastizität beziehungsweise Reduzierung der (Mooney-)Viskosität von Kautschuken dar. Die Mastikation wird durchgeführt durch Behandlung insbesondere von Naturkautschuk in Knetern oder zwischen Walzen bei möglichst niedrigen Temperaturen in Gegenwart von Mastikationshilfsmitteln (Mastizierhilfsmittel). Die dabei einwirkenden hohen mechanischen Kräfte führen zu einem "Zerreißen" der Kautschuk-Moleküle unter Bildung von Makroradikalen, deren Rekombination durch Reaktion mit Luftsauerstoff verhindert wird. Mastikationshilfsmittel wie aromatische oder heterocyclische Mercaptane beziehungsweise deren Zink-Salze oder Disulfide beschleunigen durch Begünstigung der Bildung von Primärradikalen den Mastikationsprozeß. Aktivatoren wie Metall- (Eisen-, Kupfer-, Cobalt-) Salze von Tetraazaporphyrinen oder Phthalocyaninen ermöglichen eine Erniedrigung der Mastikationstemperatur. Mastikationshilfsmittel werden bei der Mastikation von Naturkautschuk in Mengen von ca. 0,1 bis 0,5 Gew.-% in Form von Masterbatches eingesetzt, die eine gleichmäßige Verteilung dieser geringen Chemikalienmenge in der Kautschukmasse erleichtern.

Die Mastikation ist streng zu unterscheiden von dem sich bei allen üblichen lösungsmittelfreien Polymertechnologien wie Compoundieren, Fördern und Beschichten in der Schmelze ergebenden Abbau, der Degradation (engl.: degradation).

Die Degradation ist eine Kollektivbezeichnung für unterschiedliche Prozesse, die das Aussehen und die Eigenschaften von Kunststoffen verändern. Degradation kann zum Beispiel durch chemische, thermische, oxidative, mechanische oder biologische Einflüsse oder auch durch Strahleneinwirkung (wie (UV-)Licht) verursacht werden. Folge sind zum Beispiel Oxidation, Kettenspaltungen, Depolymerisation, Vernetzung beziehungsweise Abspaltung von Seitengruppen der Polymeren. Die Stabilität von Polymeren gegenüber einer Degradation kann durch Additive, zum Beispiel durch Zusatz von Stabilisatoren wie Antioxidantien oder Photostabilisatoren erhöht werden.

Verschiedene Wege zur lösungsmittelfreien Herstellung und Verarbeitung von Kautschuk-Haftklebern wurden beschrieben.

In dem Patent CA 698 518 wird ein Prozeß beschrieben, wie eine Masseherstellung durch Zusatz hoher Weichmacheranteile und/oder gleichzeitig starker Mastikation des Kautschuks zu erreichen ist. Zwar lassen sich nach diesem Verfahren Haftkleber mit extrem hoher Anfaßklebkraft erzielen, durch den relativ hohen Weichmacheranteil oder auch den starken Abbau der Molekularstruktur des Elastomers auf ein Molekulargewichtsmittel von M_{w} ≤ 1 Million ist die anwendergerechte Scherfestigkeit auch mit anschließender höherer Vernetzung nur eingeschränkt zu erreichen.

Die Verwendung von Polymerblends, wo neben nicht-thermoplastischem Naturkautschuk auch Blockcopolymere im Verhältnis von ca. 1:1 eingesetzt werden, stellt im wesentlichen eine unbefriedigende Kompromißlösung dar, da weder hohe Scherfestigkeiten beim Einsatz der Selbstklebebänder bei höheren Temperaturen, noch deutliche Verbesserungen gegenüber der im Patent beschriebenen Eigenschaften ergibt.

Der Einsatz von ausschließlich nicht-thermoplastischen Kautschuken als Elastomerkomponente in der Haftkleberrezeptierung mit dem bestehenden Kostenvorteil, den zum Beispiel Naturkautschuke gegenüber den handelsüblichen Blockcopolymeren aufweisen, und den herausragenden Eigenschaften, insbesondere der Scherfestigkeit des Naturkautschuks und entsprechender Synthesekautschuke, wird auch in den Patenten WO 94 11 175 A1, WO 95 25 774 A1, WO 97 07 963 A1 und entsprechend US 5,539,033, US 5,550,175 ausführlich dargestellt.

Hierbei werden die in der Haftklebertechnik gebräuchlichen Zusätze wie Tackifier-Harze, Weichmacher und Füllstoffe beschrieben.

Das jeweils offenbarte Herstellungsverfahren basiert auf einem Doppelschneckenextruder, der bei der gewählten Prozeßführung über Mastikation des Kautschuks und anschließender stufenweiser Zugabe der einzelnen Zusätze mit einer entsprechenden Temperaturführung die Compoundierung zu einer homogenen Haftkleberabmischung ermöglicht.

Ausführlich wird der dem eigentlichen Herstellprozeß vorgeschaltete Mastikationsschritt des Kautschuks beschrieben. Er ist notwendig und charakteristisch für das gewählte Verfahren, da er bei der dort gewählten Technologie unumgänglich für die nachfolgende Aufnahme der weiteren Komponenten und für die Extrudierbarkeit der fertig abgemischten Masse ist. Beschrieben wird auch die Einspeisung von Luftsauerstoff, wie sie von R. Brzoskowski, J.L. und B. Kalvani in Kunststoffe 80 (8), (1990), S. 922 ff., empfohlen wird, um die Kautschukmastikation zu beschleunigen.

Diese Verfahrensweise macht den nachfolgenden Schritt der Härtung durch Elektronenstrahlvernetzung (ESH) unumgänglich, ebenso wie den Einsatz von reaktiven Substanzen als ESH-Promotoren zum Erzielen einer effektiven Vernetzungsausbeute.

Beide Verfahrensschritte sind in den genannten Dokumenten beschrieben, die gewählten ESH-Promotoren neigen aber auch zu unerwünschten chemischen Vernetzungsreaktionen unter erhöhten Temperaturen, dies limitiert den Einsatz bestimmter klebrigmachender Harze und den Einsatz der erzeugten Selbstklebemassen insbesondere für pharmazeutische Anwendungen.

Die thermoplastischen Eigenschaften der erfindungsgemäßen Klebemassen erlauben eine mastikationsfreie Herstellung derselben. Als besonders vorteilhaft hierfür hat sich die Verwendung eines Doppelschneckenextruders mit mindestens einer Dosieröffnung, vorzugsweise zwischen zwei und sieben, und mindestens einer Entgasungsöffnung als kontinuierlich arbeitendes Aggregat herausgestellt. Der Doppelschneckenextruder ermöglicht kurze Mischzeiten und damit eine besonders schonende Verarbeitung der Masse, insbesondere thermisch sensibler Einsatzkomponenten.

Die Herstellung der erfindungsgemäßen Selbstklebemasse kann beispielsweise nach verschiedenen Verfahrensvarianten erfolgen. Im Compoundierungsschritt wird die erfindungsgemäße Klebmasse aus Styrolblockcopolymeren, einem oder mehreren pharmazeutischen Wirkstoffen und den benötigten Zuschlagstoffen wie niedermolekulares SBC, Füllstoffe, Weichmacher, Tackifier, Harze und/oder Additive bevorzugt in einem Doppelschneckenextruder lösungsmittelfrei hergestellt. Die pharmazeutischen Wirkstoffe können dabei unmittelbar zu Beginn des Verfahrens zugesetzt werden, können aber auch - je nach Empfindlichkeit des Wirkstoffes - erst zu einem späteren Zeitpunkt in den Doppelschneckenextruder gegeben werden. Die Zugabe kann in reiner oder gelöster Form erfolgen.

### Variante A

a) Vorlage eines Vorbatches aus SBC, gegebenenfalls einem Trennhilfsmittel und mindestens einem pharmazeutischen Wirkstoff in den Füllteil des Aggregats, gegebenenfalls Vorlage von niedermolekularem SBC, Füllstoffen, Weichmachern, Tackifiern, Harzen und/oder Additiven,
b) Übergabe der Vorlagekomponenten der Selbstklebemasse aus dem Füllteil in den Compoundierteil,
c) gegebenenfalls Zugabe der nicht in dem Füllteil aufgegebenen Komponenten der Selbstklebemasse in den Compoundierteil wie niedermolekulares SBC, Füllstoffe, Weichmacher, Tackifier Harzen und/oder Additiven,
d) gegebenenfalls Zugabe von weiteren pharmazeutischen Wirkstoffen in den Compoundierteil des Aggregats und
e) Herstellung einer homogenen Selbstklebemasse im Compoundierteil. Weiterhin kann die Herstellung der erfindungsgemäßen Selbstklebemassen beispielsweise gemäß nachfolgender Variante erfolgen.

### Variante B

a) Vorlage von SBC, gegebenenfalls mit einem Trennhilfsmittel in den Füllteil des Aggregats, gegebenenfalls Vorlage von niedermolekularem SBC, Füllstoffen, Weichmachern, Tackifiern Harzen und/oder Additiven,
b) Übergabe der Vorlagekomponenten der Selbstklebemasse aus dem Füllteil in den Compoundierteil,
c) Zugabe von mindestens einem pharmazeutischen Wirkstoff in den Compoundierteil und gegebenenfalls Zugabe der nicht in dem Füllteil aufgegebenen Komponenten der Selbstklebemasse in den Compoundierteil wie niedermolekulares SBC, Füllstoffe, Weichmacher, Tackifier Harzen und/oder Additiven und
d) Herstellung einer homogenen Selbstklebemasse im Compoundierteil.

Die Zugabe von mindestens einem pharmazeutischen Wirkstoff in den Compoundierteil und die gegebenenfalls erforderliche Zugabe der nicht in dem Füllteil aufgegebenen Komponenten der Selbstklebemasse in den Compoundierteil wie niedermolekulares SBC, Füllstoffe, Weichmacher, Tackifier, Harzen und/oder Additiven kann über die gesamte Länge des Compoundierteils erfolgen. Insbesondere sind mehrere Zudosierungsstellen möglich, so dass jede einzelne der Komponenten je nach Anforderung an die Prozeßführung gezielt über einen eigenen Zulauf zudosiert werden kann.

Weiterhin sollte im Aggregat eine Temperatur von 85 - 120 °C eingehalten werden, bevorzugt 85 - 110 C, besonders bevorzugt 85 - 100 °C, um eine thermische Schädigung insbesondere des oder der Wirkstoffe auszuschließen.

In einer vorteilhaften Ausführungsform des Verfahrens ist zwischen Aggregat und Beschichtungsvorrichtung eine Schmelzepumpe oder ein Extruder zur Förderung der Selbstklebemassen angeordnet.

Im zweiten, vorteilhafterweise im Verbund mit dem Compoundierschritt im Doppelschneckenextruder erfolgenden Verfahrensschritt zur Herstellung der erfindungsgemäßen wirkstoffhaltigen Pflastersysteme wird die erfindungsgemäß hergestellte Selbstklebemasse mit einem Auftragswerk auf einen bahnförmigen Träger, auf eine Trennfolie oder auf ein Trennpapier lösemittelfrei beschichtet. Die Beschichtung kann vollflächig, aber auch partiell erfolgen.

Um einen definierten, luftblasenfreien Masseauftrag auf dem bahnförmigen Material zu erhalten, ist es vorteilhaft, dass die Selbstklebemasse vor Eintritt in die Beschichtungseinheit einer Entgasung unterworfen wird, was besonders wichtig ist im Falle der Verwendung von Schutzgasen während des Compoundierprozesses im Doppelschneckenextruder.

Zusätzlich kann eine Entgasung unter Einfluss von Unterdruck oder gegen die Atmosphäre vorzugsweise in Schneckenmaschinen erfolgen.

Zur Beschichtung auf bahnförmige Materialien eignen sich verschiedene Verfahren. Lösungsmittelfreie Selbstklebemassen lassen sich mittels einer dem Doppelschneckenextruder nachgeschalteten Extrusionsdüse beschichten. Zum Druckaufbau für die Düsenbeschichtung werden Einschneckenextruder und/oder Schmelzepumpen besonders bevorzugt, so dass die Beschichtung der bahnförmigen Trägermaterialien mit Masseaufträgen sehr geringer Schwankungsbreite erfolgen kann.

Eine weitere Möglichkeit zur Beschichtung von bahnförmigen Trägermaterialien mit der nach erfindungsgemäßen Verfahren hergestellten wirkstoffhaltigen Selbstklebemasse ist die Verwendung von Walzenbeschichtungsauftragswerken oder Mehrwalzen-Beschichtungskalandern, die vorzugsweise aus mindestens zwei Beschichtungswalzen bestehen, wobei die Selbstklebemasse bei Durchgang durch einen oder mehrere Walzenspalte vor Übergabe auf das bahnförmige Material auf die gewünschte Dicke geformt wird. Dieses Beschichtungsverfahren wird besonders dann bevorzugt, wenn eine Beschichtung mit Extrusionsdüsen allein nicht mehr die erforderte Genauigkeit im Masseauftrag liefert.

Je nach Art des zu beschichtenden bahnförmigen Trägermaterials kann die Beschichtung im Gleichlauf- oder Gegenlaufverfahren erfolgen.

Die Beschichtung ist auf Walzenbeschichtungsauftragswerken oder Mehrwalzen-Beschichtungskalandern bei Temperaturen unterhalb von 120°C möglich, so dass auch Selbstklebemassen beschichtet werden können, die thermisch empfindliche Wirkstoffe enthalten. Zum Zwecke erhöhter Gasblasenfreiheit der beschichteten Klebmasse kann zwischen Doppelschneckenextruder und Auftragswerk eine Vakuumentgasung, zum Beispiel eine Vakuumkammer, ein Entgasungsextruder, Luftrakel oder ähnliches installiert sein.

Durch dieses Verfahren erfolgt keine eigenschaftsschädigende Mastikation des SBC, da kurz nach dessen Vorlage das Aufschmelzen der physikalisch vernetzten Styroldomänen erfolgt.

Der Doppelschneckenextruder verfügt über einen oder vorzugsweise mehrere separate Temperier- beziehungsweise Kühlkreise, um ein Temperaturregime zu ermöglichen, das den Einsatz thermisch empfindlicher pharmazeutischer Wirkstoffe erlaubt. In Fällen, wo dies nicht erforderlich ist, können die Temperierkreise auch miteinander verbunden werden, um die Anzahl an Temperiergeräten möglichst gering zu halten.

Dieses Verfahren erlaubt die Herstellung von erfindungsgemäßen Selbstklebemassen mit pharmazeutischen Wirkstoffen und insbesondere durch die Verwendung eines Extruders im Verbund mit einer nachgeschalteten Beschichtungseinheit zur Herstellung von selbstklebend ausgerüsteten Artikeln, die ihrerseits zur Herstellung von Pflastern oder Binden eingesetzt werden, unter Erlangung besonderer Kostenvorteile.

Die erfindungsgemäßen Klebemassen werden im wesentlichen unter den bereits dargelegten Verfahrensschritten hergestellt, welche optional unter einer Schutzgasatmosphäre zur Vermeidung von oxidativem Polymerabbau durchgeführt werden können.

Die Matrix zeichnet sich aus durch hervorragende Hafteigenschaften auf der Haut, durch eine leichte und schmerzfreie Wiederablösbarkeit, sowie vor allem durch ihr äußerst geringes Potential, Hautreizungen hervorzurufen. Der Herstellungsprozess verläuft unter vollständigem Verzicht von Lösungsmitteln.

Gegebenfalls kann die offene, auf die Haut zu applizierende Klebseite mit einer wiederablösbaren, abdeckenden Schutzschicht eingedeckt sein.

### Beispiel 1

Zur kontinuierlichen lösungsmittelfreien Herstellung eines Prototypen einer mit Wirkstoff dotierten Selbstklebemasse diente ein Doppelschneckenextruder, wobei die folgenden Beispielrezeptur (alle Angaben in Teilen pro Hundert (phr) bezogen auf die Summe des Blockcopolymernanteils) angewendet wurde:
A) 100,0 phr A-B/A-B-A Blockcopolymer, bestehend aus harten und weichen Segmenten besteht, mit einem Verhältnis von A-B-A zu A-B von 29:21 und einem Styrolgehalt von 15 Massen-% (Vector 4114, Dexco)
C) 33,0 phr Polyterpenharz (Sylvares TR 7115, Arizona Chemical)
D) 41,9 phr Kolophoniumesterharz (Foral 90, Eastman)
E) 25,6 phr Kohlenwasserstoffharz (Wingtack 95, Goodyear)
F) 23,6 phr Mineralöl (Whitemor WOM 14, Castrol Ltd.)
H) 8,9 phr Alterungsschutzmittel (Irganox 1010, Ciba Specialty Chemicals)
I) 259,6 phr Salicylsäure DAB als Wirkstoff

### Beispiel 2

Eine dotierte Selbstklebemasse wurde wie in Beispiel 1 nach der folgenden Beispielrezeptur (alle Angaben in Teilen pro Hundert (phr) bezogen auf die Summe des Blockcopolymernanteils) hergestellt:
A) 100,0 phr A-B/A-B-A Blockcopolymer, bestehend aus harten und weichen Segmenten, mit einem Verhältnis von A-B-A zu A-B von 83:17 und einem Styrolgehalt von 15 Massen-% (Kraton D-1107, Kraton)
C) 67,7 phr Polyterpenharz (Sylvares TR 7115, Arizona Chemical)
D) 86,6 phr Kohlenwasserstoffharz (Wingtack 95, Goodyear)
F) 57,1 phr Wollwachs DAB
H) 11,5 phr Alterungsschutzmittel (Lowinox 22M46, Great Lakes Chemical Corp.)
I) 226,4 phr Salicylsäure DAB als Wirkstoff

### Beispiel 3

Für die kontinuierliche lösungsmittelfreie Herstellung eines weiteren Prototypen einer mit Wirkstoff dotierten Selbstklebemasse wurde wie in Beispiel 1 vorgegangen. Hierbei wurde die folgende Beispielrezeptur (alle Angaben in Teilen pro Hundert (phr) bezogen auf die Summe des Blockcopolymernanteils) verwendet:
A) 65,3 phr A-B-A Blockcopolymer, bestehend aus harten und weichen Segmenten besteht, mit einem Styrolgehalt von 15 Massen-% (Vector 4114, Dexco)
B) 34,7 phr A-B/A-B-A Blockcopolymer, bestehend aus harten und weichen Segmenten besteht, mit einem Verhältnis von A-B-A zu A-B von 41:9 und einem Styrolgehalt von 15 Massen-% (Vector 4113, Dexco)
C) 74,3 phr Kolophoniumesterharz (Foral 90, Eastman)
F) 28,1 phr Bienenwachs DAB
G) 173,2 phr Wollwachs DAB
H) 14,5 phr Alterungsschutzmittel (Lowinox 22M46, Great Lakes Chemical Corp.)
I) 551,2 phr Salicylsäure DAB als Wirkstoff

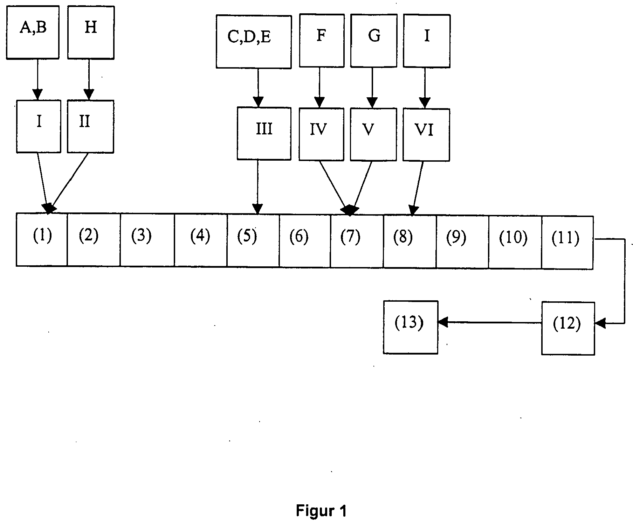

In der Figur 1 ist eine schematische Übersicht über die zur Durchführung des Verfahrens verwendete Anlage gezeigt.

Die Rohstoffe A, B und H wurden jeweils über eine gravimetrische Dosierung (I) und (II) dem Füllteil eines Doppelschneckenextruders zugeführt.

Das Material wurde über eine erste fördernde Verfahrenszone (1) weiteren Zonen (2) - (4) zugeführt, die das Material mischten.

In die fördernde Verfahrenszone (5) wurden - rezepturabhängig - die Komponenten C/D/E gravimetrisch zudosiert (III). Anschließend wurde gemischt und gefördert (6). Danach folgte die Zone (7), die das Material förderte und der die Komponenten F/G über volumetrisch arbeitende Zahnradpumpen (IV) und (V) zudosiert wurden. Im Anschluß daran wurde das Material wieder gemischt.

Danach folgte die Zone (8), die das Material förderte und der die Komponente I über eine gravimetrische Dosierung (VI) zudosiert wurde.

In den Zonen (9) - (11) wurde das Material gemischt und gefördert.

Anschließend wurde die Selbstklebemasse über eine 350mm-Breitschlitzdüse (12) ausgeformt und ausgetragen. In einem Glättwerk (13) wurde kalandriert und mit zwei PET-Folien kaschiert.

Die Drehzahl des Extruders betrug zwischen 100 und 150 rpm. Am Austritt aus dem Extruder hatte die Masse eine Temperatur zwischen 90 °C und 100 °C.

## Patentansprüche

1. Wirkstoffhaltiges Matrixpflaster zur kontrollierten Abgabe von mindestens einem pharmazeutischen Wirkstoff an die Haut, bestehend aus einer flexiblen Deckschicht und einer wirkstoffhaltigen, wasserunlöslichen haftklebrigen Matrix, **dadurch gekennzeichnet, dass** die Matrix mindestens ein Polymer auf Basis eines Styrolblockcopolymeren (SBC) sowie einen Anteil mindestens eines pharmazeutischen Wirkstoffes von mindestens 34 Gew.-%, bezogen auf die Gesamtmatrixmasse, enthält.

2. Wirkstoffhaltiges Matrixpflaster nach Anspruch 1, **dadurch gekennzeichnet, dass** die SBC-Polymere A-B- oder A-B-A-Blockcopolymer oder deren Mischungen, wobei die harte Phase A vornehmlich Polystyrol oder dessen Derivate und die weiche Phase B Ethylen, Propylen, Butylen, Butadien, Isopren oder deren Mischungen enthält.

3. Wirkstoffhaltiges Matrixpflaster nach Anspruch 2, **dadurch gekennzeichnet, dass** der Styrolgehalt der verwendeten Blockcopolymeren weniger als 35 Gew.-% beträgt.

4. Wirkstoffhaltiges Matrixpflaster nach einem der Ansprüche 1, 2 oder 3, **dadurch gekennzeichnet, dass** der pharmazeutische Wirkstoff aus der Gruppe der lokal wirksamen Wirkstoffe entstammt.

5. Wirkstoffhaltiges Matrixpflasters nach einem der Ansprüche 1 bis 4, **dadurch gekennzeichnet, dass** alle Komponenten der haftklebrigen Matrix unter Verzicht auf den Zusatz von Lösungsmittel in der Schmelze homogenisiert worden sind.

6. Verfahren zur kontinuierlichen lösungsmittelfreien und mastikationsfreien Herstellung von Selbstklebemassen auf Basis von SBC mit mindestens einem pharmazeutischen Wirkstoff in einem kontinuierlich arbeitenden Aggregat mit einem Füll- und einem Compoundierteil, umfassend
a) Vorlage eines Vorbatches aus SBC, gegebenenfalls mit einem Trennhilfsmittel, und mindestens einem pharmazeutischen Wirkstoff mit einem Anteil von mindestens 34-Gew.%, bezogen auf die Gesamtselbstklebemasse, in den Füllteil des Aggregats,
gegebenenfalls Vorlage von niedermolekularem SBC, Füllstoffen, Weichmachern, Tackifiern, Harzen und/oder Additiven,
b) Übergabe der Vorlagekomponenten der Selbstklebemasse aus dem Füllteil in den Compoundierteil,
c) gegebenenfalls Zugabe der nicht in dem Füllteil aufgegebenen Komponenten der Selbstklebemasse in den Compoundierteil wie niedermolekulares SBC, Füllstoffe, Weichmacher, Harze und/oder Additive,
d) gegebenenfalls Zugabe von weiteren pharmazeutischen Wirkstoffen in den Compoundierteil des Aggregats und
e) Herstellung einer homogenen Selbstklebemasse im Compoundierteil.

7. Verfahren zur kontinuierlichen lösungsmittelfreien und mastikationsfreien Herstellung von Selbstklebemassen auf Basis von SBC mit mindestens einem pharmazeutischen Wirkstoff mit einem Anteil von mindestens 34-Gew.%, bezogen auf die Gesamtselbstklebemasse, in einem kontinuierlich arbeitenden Aggregat mit einem Füll- und einem Compoundierteil, umfassend
a) Vorlage von SBC, gegebenenfalls mit einem Trennhilfsmittel in den Füllteil des Aggregats, gegebenenfalls Vorlage von niedermolekularem SBC, Füllstoffen, Weichmachern, Tackifiern, Harzen und/oder Additiven,
b) Übergabe der Vorlagekomponenten der Selbstklebemasse aus dem Füllteil in den Compoundierteil,
c) Zugabe von mindestens einem pharmazeutischen Wirkstoff in den Compoundierteil und gegebenenfalls Zugabe der nicht in dem Füllteil aufgegebenen Komponenten der Selbstklebemasse in den Compoundierteil wie niedermolekulares SBC, Füllstoffe, Weichmacher Tackifier, Harze und/oder Additive und
d) Herstellung einer homogenen Selbstklebemasse im Compoundierteil.

8. Verfahren zur Herstellung von Selbstklebemassen auf Basis von SBC mit mindestens einem pharmazeutischen Wirkstoff mit einem Anteil von mindestens 34-Gew.% nach einem der Ansprüche 6 bis 7, **dadurch gekennzeichnet, dass** alle Komponenten in einem kontinuierlichen oder diskontinuierlichen Prozess bei einer Temperatur von 85-120 °C, bevorzugt von 85-110 °C, besonders bevorzugt von 85-100 °C verarbeitet werden.

9. Verfahren zur Herstellung von Selbstklebemassen auf Basis von SBC mit mindestens einem pharmazeutischen Wirkstoff mit einem Anteil von mindestens 34-Gew.% nach einem der Ansprüche 6 bis 8, **dadurch gekennzeichnet, dass** das Aggregat ein Doppelschneckenextruder mit mindestens einer, vorzugsweise zwischen zwei und sieben, Dosieröffnung und mindestens einer Entgasungsöffnung ist.

10. Verfahren zur Herstellung von Selbstklebemassen auf Basis von SBC mit mindestens einem pharmazeutischen Wirkstoff mit einem Anteil von mindestens 34-Gew.% nach einem der Ansprüche 6 bis 9, **dadurch gekennzeichnet, dass** zwischen Aggregat und Beschichtungsvorrichtung eine Schmelzepumpe oder ein Extruder zur Förderung der Selbstklebemasse angeordnet ist.

11. Verfahren zur Herstellung eines wirkstoffhaltigen Matrixpflasters durch Herstellung von Selbstklebemassen auf Basis von SBC mit mindestens einem pharmazeutischen Wirkstoff mit einem Anteil von mindestens 34-Gew%. nach einem der Ansprüche 6 bis 10, **dadurch gekennzeichnet, dass** die Selbstklebemasse auf ein bahnförmiges Material, auf eine Trennfolie oder auf ein Trennpapier beschichtet wird und gegebenenfalls mit einem weiteren Abdeckmaterial eingedeckt wird.

12. Verfahren zur Herstellung eines wirkstoffhaltigen Matrixpflasters nach Anspruch 11, **dadurch gekennzeichnet, dass** die Rohstoffe in den Füllteil und/oder in den Compoundierteil eines Aggregates zugegeben werden, homogenisiert werden und aus dem Aggregat ausgetragen werden.

13. Verfahren zur Herstellung eines wirkstoffhaltigen Matrixpflasters nach dem Anspruch 11 oder 12, **dadurch gekennzeichnet, dass** die Beschichtung des bahnförmigen Materials mit einer Extrusionsdüse erfolgt.

14. Verfahren zur Herstellung eines wirkstoffhaltigen Matrixpflasters nach dem Anspruch 11 oder 12, **dadurch gekennzeichnet, dass** die Beschichtung des bahnförmigen Materials mit einem Walz- oder Kalanderwerk erfolgt, wobei die Selbstklebemasse bei Durchgang durch einen oder mehrere Walzenspalte auf die gewünschte Dicke geformt wird.

15. Verfahren zur Herstellung eines wirkstoffhaltigen Matrixpflasters nach dem Anspruch 11 oder 12, **dadurch gekennzeichnet, dass** die Beschichtung des bahnförmigen Materials mit einer Extrusionsdüse und einem Walz- oder Kalanderwerk erfolgt, wobei die Selbstklebemasse bei Durchgang durch einen oder mehrere Walzenspalte auf die gewünschte Dicke geformt wird.

16. Verfahren zur Herstellung eines wirkstoffhaltigen Matrixpflasters nach einem der Ansprüche 11 bis 15, **dadurch gekennzeichnet, dass** die Dicke der Selbstklebemasse auf dem bahnförmigen Material zwischen 10 µm und 2000 µm beträgt, vorzugsweise zwischen 100 µm und 500 µm.

17. Wirkstoffhaltiges Matrixpflaster mit einem Wirkstoffanteil von mindestens 34 Gew.% erhältlich nach einem der Ansprüche 11 bis 16.

## Claims

1. Active ingredient matrix plaster for the controlled delivery to the skin of at least one pharmaceutical active ingredient, composed of a flexible outer layer and a pressure-sensitively adhesive, water-insoluble, active ingredient matrix, **characterized in that** the matrix comprises at least one styrene block copolymer (SBC)-based polymer and also a fraction of at least 34 wt.% of at least one pharmaceutical active ingredient, based on the total matrix mass.

2. Active ingredient matrix plaster according to Claim 1, **characterized in that** the SBC polymers A-B or A-B-A block copolymer or mixtures thereof, the hard phase, A, comprising principally polystyrene or its derivatives and the soft phase, B, comprising ethylene, propylene, butylene, butadiene, isoprene or mixtures thereof.

3. Active ingredient matrix plaster according to Claim 2, **characterized in that** the styrene content of the block copolymers used is less than 35 wt.%.

4. Active ingredient matrix plaster according to any one of Claims 1, 2 or 3, **characterized in that** the pharmaceutical active ingredient comes from the group of locally active ingredients.

5. Active ingredient matrix plaster according to any one of Claims 1 to 4, **characterized in that** all components of the pressure-sensitively adhesive matrix have been homogenized in the melt without the addition of solvent.

6. Process for the continuous solvent-free and mastication-free preparation of self-adhesive compositions based on SBC with at least one pharmaceutical active ingredient in a continuously operating apparatus having a feed section and a compounding section, comprising
a) feeding an initial batch comprising SBC, optionally with a release auxiliary, and at least one pharmaceutical active ingredient with a fraction of at least 34 wt.%, based on the total self-adhesive composition, into the feed section of the apparatus,
if desired, feeding low molecular mass SBC, fillers, plasticizers, tackifiers, resins and/or additives,
b) transferring the feed components of the self-adhesive composition from the feed section to the compounding section,
c) if desired, adding the components of the self-adhesive composition that have not been introduced in the filling section, such as low molecular mass SBC, fillers, plasticizers, resins and/or additives, to the compounding section,
d) if desired, adding further pharmaceutical active ingredients to the compounding section of the apparatus, and
e) preparing a homogeneous self-adhesive composition in the compounding section.

7. Process for the continuous solvent-free and mastication-free preparation of self-adhesive compositions based on SBC with a fraction of at least 34 wt.% of at least one pharmaceutical active ingredient, based on the total self-adhesive composition, in a continuously operating apparatus having a feed section and a compounding section, comprising
a) feeding SBC, optionally with a release auxiliary, into the feed section of the apparatus if desired, feeding low molecular mass SBC, fillers, plasticizers, tackifiers, resins and/or additives,
b) transferring the feed components of the self-adhesive composition from the feed section to the compounding section,
c) adding at least one pharmaceutical active ingredient to the compounding section and, if desired, adding the components of the self-adhesive composition that have not been introduced in the filling section, such as low molecular mass SBC, fillers, plasticizers, tackifiers, resins and/or additives, to the compounding section, and
d) preparing a homogeneous self-adhesive composition in the compounding section.

8. Process for preparing self-adhesive compositions based on SBC with at least one pharmaceutical active ingredient with a fraction of at least 34 wt.%, according to any one of Claims 6 to 7, **characterized in that** all components are processed in a continuous or batchwise operation at a temperature of 85-120°C, preferably at 85-110°C, more preferably at 85-100°C.

9. Process for preparing self-adhesive compositions based on SBC with at least one pharmaceutical active ingredient with a fraction of at least 34 wt.%, according to any one of Claims 6 to 8, **characterized in that** the apparatus is a twin-screw extruder having at least one, preferably between two and seven, metering port and at least one devolatilization port.

10. Process for preparing self-adhesive compositions based on SBC with at least one pharmaceutical active ingredient with a fraction of at least 34 wt.%, according to any one of Claims 6 to 9, **characterized in that** a melt pump or an extruder for conveying the self-adhesive composition is arranged between the apparatus and the coating device.

11. Process for producing an active ingredient matrix plaster by preparing self-adhesive compositions based on SBC with at least one pharmaceutical active ingredient with a fraction of at least 34 wt.%, according to any one of Claims 6 to 10, **characterized in that** the self-adhesive composition is coated onto a material in web form, onto a release film or onto a release paper and if desired is enveloped with a further liner material.

12. Process for producing an active ingredient matrix plaster according to Claim 11, **characterized in that** the raw materials are added to the feed section and/or to the compounding section of an apparatus and are homogenized and discharged from the apparatus.

13. Process for producing an active ingredient matrix plaster according to Claim 11 or 12, **characterized in that** the coating of the material in web form takes place with an extrusion die.

14. Process for producing an active ingredient matrix plaster according to Claim 11 or 12, **characterized in that** the coating of the material in web form takes place with a roll mill or calender mill, the self-adhesive composition being shaped to the desired thickness as it passes through one or more roll nips.

15. Process for producing an active ingredient matrix plaster according to Claim 11 or 12, **characterized in that** the coating of the material in web form takes place with an extrusion die and with a roll mill or calender mill, the self-adhesive composition being shaped to the desired thickness as it passes through one or more roll nips.

16. Process for producing an active ingredient matrix plaster according to any one of Claims 11 to 15, **characterized in that** the thickness of the self-adhesive composition on the material in web form is between 10 µm and 2000 µm, preferably between 100 µm and 500 µm.

17. Active ingredient matrix plaster having an active ingredient fraction of at least 34 wt.%, obtainable according to any one of Claims 11 to 16.

## Revendications

1. Timbre matriciel contenant une substance active pour la libération contrôlée d'au moins une substance active pharmaceutique sur la peau, constitué par une couche supérieure flexible et une matrice adhésive de contact insoluble dans l'eau, contenant une substance active, **caractérisé en ce que** la matrice contient au moins un polymère à base d'un copolymère séquencé de styrène (SBC), ainsi qu'une proportion d'au moins une substance active pharmaceutique d'au moins 34 % en poids, par rapport à la masse totale de la matrice.

2. Timbre matriciel contenant une substance active selon la revendication 1, **caractérisé en ce que** les polymères SBC un copolymère séquencé A-B ou A-B-A ou leurs mélanges, la phase dure A contenant principalement du polystyrène ou ses dérivés et la phase molle B contenant de l'éthylène, du propylène, du butylène, du butadiène, de l'isoprène ou leurs mélanges.

3. Timbre matriciel contenant une substance active selon la revendication 2, **caractérisé en ce que** la teneur en styrène des copolymères séquencés utilisés est de moins de 35 % en poids.

4. Timbre matriciel contenant une substance active selon l'une quelconque des revendications 1, 2 ou 3, **caractérisé en ce que** la substance active pharmaceutique est issue du groupe des substances actives à effet local.

5. Timbre matriciel contenant une substance active selon l'une quelconque des revendications 1 à 4, **caractérisé en ce que** tous les composants de la matrice adhésive de contact ont été homogénéisés à l'état fondu, en renonçant à l'ajout de solvants.

6. Procédé de fabrication continue sans solvant et sans mastication de masses auto-adhésives à base de SBC contenant au moins une substance active pharmaceutique dans un appareil fonctionnant en continu muni d'une partie de remplissage et d'une partie de mélange, comprenant
a) le chargement initial d'un lot préalable de SBC, éventuellement avec un adjuvant de démoulage, et d'au moins une substance active pharmaceutique en une proportion d'au moins 34 % en poids, par rapport à la masse auto-adhésive totale, dans la partie de remplissage de l'appareil,
éventuellement le chargement initial de SBC de faible poids moléculaire, de charges, de plastifiants, d'agents collants, de résines et/ou d'additifs,
b) le transfert des composants de la charge initiale de la masse auto-adhésive de la partie de remplissage vers la partie de mélange,
c) éventuellement l'ajout des composants de la masse auto-adhésive non introduits dans la partie de remplissage dans la partie de mélange, tels que du SBC de faible poids moléculaire, des charges, des plastifiants, des résines et/ou des additifs,
d) éventuellement l'ajout d'autres substances actives pharmaceutiques dans la partie de mélange de l'appareil, et
e) la fabrication d'une masse auto-adhésive homogène dans la partie de mélange.

7. Procédé de fabrication continue sans solvant et sans mastication de masses auto-adhésives à base de SBC contenant au moins une substance active pharmaceutique en une proportion d'au moins 34 % en poids, par rapport à la masse auto-adhésive totale, dans un appareil fonctionnant en continu muni d'une partie de remplissage et d'une partie de mélange, comprenant
a) le chargement initial de SBC, éventuellement avec un adjuvant de démoulage dans la partie de remplissage de l'appareil, éventuellement le chargement initial de SBC de faible poids moléculaire, de charges, de plastifiants, d'agents collants, de résines et/ou d'additifs,
b) le transfert des composants de la charge initiale de la masse auto-adhésive de la partie de remplissage vers la partie de mélange,
c) l'ajout d'au moins une substance active pharmaceutique dans la partie de mélange et éventuellement l'ajout des composants de la masse auto-adhésive non introduits dans la partie de remplissage dans la partie de mélange, tels que du SBC de faible poids moléculaire, des charges, des plastifiants, des agents collants, des résines et/ou des additifs, et
d) la fabrication d'une masse auto-adhésive homogène dans la partie de mélange.

8. Procédé de fabrication de masses autoadhésives à base de SBC contenant au moins une substance active pharmaceutique en une proportion d'au moins 34 % en poids selon l'une quelconque des revendications 6 à 7, **caractérisé en ce que** tous les composants sont traités par un procédé continu ou discontinu à une température de 85 à 120 °C, de préférence de 85 à 110 °C, de manière particulièrement préférée de 85 à 100 °C.

9. Procédé de fabrication de masses auto-adhésives à base de SBC contenant au moins une substance active pharmaceutique en une proportion d'au moins 34 % en poids selon l'une quelconque des revendications 6 à 8, **caractérisé en ce que** l'appareil est une extrudeuse bivis comprenant au moins une, de préférence entre deux et sept, ouvertures d'alimentation et au moins une ouverture de dégazage.

10. Procédé de fabrication de masses auto-adhésives à base de SBC contenant au moins une substance active pharmaceutique en une proportion d'au moins 34 % en poids selon l'une quelconque des revendications 6 à 9, **caractérisé en ce qu'**une pompe de masse fondue ou une extrudeuse pour le transport de la masse auto-adhésive est agencée entre l'appareil et un dispositif de revêtement.

11. Procédé de fabrication d'un timbre matriciel contenant une substance active par fabrication de masses auto-adhésives à base de SBC contenant au moins une substance active pharmaceutique en une proportion d'au moins 34 % en poids selon l'une quelconque des revendications 6 à 10, **caractérisé en ce que** la masse auto-adhésive est revêtue sur un matériau en forme de bande, sur une feuille de séparation ou sur un papier de séparation, et éventuellement recouverte avec un matériau de couverture supplémentaire.

12. Procédé de fabrication d'un timbre matriciel contenant une substance active selon la revendication 11, **caractérisé en ce que** les matières premières sont introduites dans la partie de remplissage et/ou dans la partie de mélange d'un appareil, homogénéisées et déchargées de l'appareil.

13. Procédé de fabrication d'un timbre matriciel contenant une substance active selon la revendication 11 ou 12, **caractérisé en ce que** le revêtement du matériau en forme de bande a lieu avec une buse d'extrusion.

14. Procédé de fabrication d'un timbre matriciel contenant une substance active selon la revendication 11 ou 12, **caractérisé en ce que** le revêtement du matériau en forme de bande a lieu avec un laminoir ou une calandre, la masse auto-adhésive étant façonnée à l'épaisseur souhaitée par passage dans un ou plusieurs espaces entre des cylindres.

15. Procédé de fabrication d'un timbre matriciel contenant une substance active selon la revendication 11 ou 12, **caractérisé en ce que** le revêtement du matériau en forme de bande a lieu avec une buse d'extrusion et un laminoir ou une calandre, la masse auto-adhésive étant façonnée à l'épaisseur souhaitée par passage dans un ou plusieurs espaces entre des cylindres.

16. Procédé de fabrication d'un timbre matriciel contenant une substance active selon l'une quelconque des revendications 11 à 15, **caractérisé en ce que** l'épaisseur de la masse auto-adhésive sur le matériau en forme de bande est comprise entre 10 µm et 2 000 µm, de préférence entre 100 µm et 500 µm.

17. Timbre matriciel contenant une substance active ayant une proportion de substance active d'au moins 34 % en poids, pouvant être obtenu selon l'une quelconque des revendications 11 à 16.
